(19) Europäisches Patentamt
European Patent Office
Office européen des brevets

(11) **EP 4 447 907 B1**

(12) # EUROPEAN PATENT SPECIFICATION

(45) Date of publication and mention of the grant of the patent:
**22.04.2026 Bulletin 2026/17**

(21) Application number: **22813984.6**

(22) Date of filing: **07.11.2022**

(51) International Patent Classification (IPC):
***A61K 8/24*** *(2006.01)* ***A61K 8/55*** *(2006.01)*
***A61Q 11/00*** *(2006.01)*

(52) Cooperative Patent Classification (CPC):
**A61Q 11/00; A61K 8/24; A61K 8/55;** A61K 2800/43

(86) International application number:
**PCT/EP2022/080900**

(87) International publication number:
**WO 2023/110213 (22.06.2023 Gazette 2023/25)**

(54) **ORAL CARE COMPOSITION COMPRISING PHYTATES AND PIGMENT**

MUNDPFLEGEZUSAMMENSETZUNG MIT PHYTATEN UND PIGMENT

COMPOSITION DE SOINS BUCCO-DENTAIRES COMPRENANT DES PHYTATES ET UN PIGMENT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **16.12.2021 EP 21214950**

(43) Date of publication of application:
**23.10.2024 Bulletin 2024/43**

(73) Proprietors:
- **Unilever IP Holdings B.V.**
  **6708 WH Wageningen (NL)**
  Designated Contracting States:
  **AL AT BE BG CH CZ DK EE ES FI FR GR HR HU IS LI LT LU LV MC ME MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **ALLAHBASH, Shahin**
  **6708 WH Wageningen (NL)**
- **BARNE, Sameer Keshav**
  **6708 WH Wageningen (NL)**

(74) Representative: **Unilever Patent Group**
**Bronland 14**
**6708 WH Wageningen (NL)**

(56) References cited:
**WO-A1-2016/099544** **US-A1- 2007 122 358**
**US-A1- 2015 289 961**

- **DATABASE WPI Week 201930, Derwent World Patents Index; AN 2019-134678, XP002806707**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the Invention

The present invention relates to oral care compositions to whiten the teeth by removing stains.

### Background of the Invention

Colour of our teeth is influenced by their intrinsic colour and the presence of any extrinsic stains thereon. Stains are a result of adsorption of materials into the pellicle on the surface of enamel. Factors responsible for it include improper brushing techniques, smoking, consumption of coloured foods (e.g., red wine), age and the use of certain cationic agents like chlorhexidine or salts of tin and iron. The chromogens in beverages are responsible for causing dental stains and they include tannins composed of polyphenols such as catechins. These materials generate color due to the presence of conjugated double bonds and are believed to interact with the tooth surface via an ion exchange mechanism. People have strong desire for whiter teeth. This desire has given rise to a growing trend of whitening products which range from toothpastes to mouthwashes and chewing gums.

Traditional tooth whitening methods involve bleaching or removal of stains using abrasives.

Toothpastes meant for whitening rely on an optimization of abrasive and chemical components for removal of stains. During brushing, these abrasive particles get temporarily trapped between the toothbrush and the stained surface (of teeth) to abrade away the stains. Chemical ingredients may also be used, usually in conjunction with abrasive particles, and these include calcium chelators, polymers, surfactants, enzymes and oxidising agents. A variety of stain-preventing oral care compositions are known. Likewise, are known, a variety of compositions that reduce the tendency of the teeth to get stained. Peroxides can bleach teeth, remove stains, and kill cariogenic bacteria. However, peroxides are highly reactive with common ingredients found in oral care compositions. Moreover, hydrogen peroxide can spontaneously decompose to form oxygen gas and water, so that on storage, the composition containers may bloat or leak. Consequently, the remainder of the formulation will have lower whitening and cleaning efficacy.

Generally, an oral care composition, such as toothpaste, is considered efficacious if it dislodges or removes stains every single time it is used.

WO2020/135952 A1 (Unilever) discloses an oral care composition of pH 6.0 and 9.0. comprising hexametaphosphate and Pluronic® type of polymers. The weight ratio of the polymer to hexametaphosphate is from 1:4 to 4:1.

WO2021063581 A1 (Unilever) discloses an oral care spray composition comprising a Pluronic® polymer and hexametaphosphate where weight ratio of the polymer to hexametaphosphate is from 1:5 to 5:1.

US20160331652 A1 (Lotte) discloses a stain-removing chewing gum containing 0.33% by weight to 2.0% by weight of sodium metaphosphate.

WO07111616 A1 (P&G) discloses oral care compositions containing a bleaching agent and polyphosphorylated compounds having at least 3 phosphate groups such as linear condensed polyphosphate polymers and phytate, for enhanced whitening and stain prevention of teeth.

US 2020/0046621 A1 (Kao) discloses an oral composition which selectively removes deposited stains present in the interprismatic space on the tooth enamel surface layer and prevents new stains from adhering and depositing thereon. The oral composition comprises phytic acid or a salt thereof and tripolyphosphoric acid and a specific polyvalent metal or a salt thereof in a molar amount of less than 0.1 times that of phytic acid and has a pH of 5.5 to 6.5.

US2015289961 A1 (Kao) discloses an adhesive sheet for teeth comprising a layer (A) that has a pH of 6.6 to 10.5 and a layer (B) that comprises phytic acid or hexamethaphosphoric acid, an organic acid having a pKa of from 4.5 to 7.0. The pH of layer B is from 3.5 to 6.5.

EP2741732 A1 (P&G, 2014)) discloses an oral care composition comprising a stannous ion source a zinc ion source, a phytic acid or a phytic acid salt, characterised in that the phytic acid or phytic acid salt has an average IPn of greater than 5.2. Stains and astringency, typically associated with toothpastes, are avoided.

EP1935395 A1 (Unilever) discloses from 0.01 to 0.3% by weight of a pigment having a hue angle, h, in the CIELAB system of from 220 to 320 degrees, characterized in that the composition further comprises a soluble deposition aid for said pigment.

US2015289961 A1 (Kao) discloses an adhesive sheet for whitening effect and gloss which needs to be attached to teeth. The sheet has a layer (A) of pH 6.6 to 10.5 and a layer (B) having phytic acid, hexamethaphosphoric acid, or an organic acid of pKa 4.5 to 7. The pH of this layer is 3.5 to 6.5 or.

US2007122358 A1 (P&G) discloses an abrasive-free having hydrophilic clay material, phytic acid compound, an oral care active, and a polar solvent carrier.

WO2016099544 A1 (Colgate) discloses a tooth whitening oral care having flakes of a water soluble whitening film, a dye with a hue angle in the CIELAB system ranging from 200 to 320 degrees, and an orally acceptable carrier vehicle. The flakes contain a film-forming polymer comprising hydroxyalkyl cellulose wherein the hydroxyalkyl cellulose

with viscosity of 1 to 1000 mPa.s.

CN109223642 A (Cai Yinzhong, 2019) discloses aqueous mouthwash containing sodium metaphosphate 20-30 parts, phytic acid 5-10 part and tin pyrophosphate 2-6 parts. The mouthwash cleans teeth, continuously refreshes breath and keeps the oral cavity clean.

However, there is need for efficacious composition that is not only effective each time it is used but also has a longer-term efficacy, i.e., the ones which not only provide stain release immediately on use but continue to provide stain repellency or anti-stain benefits.

Summary of the Invention

We have surprisingly determined that at least some of the problems can be solved by way of the present invention.

Disclosed is an oral care composition comprising:

(i) an organic polyphosphate or a water-soluble salt thereof, said polyphosphate or said salt having average chain length of at least 4;

(ii) an inorganic polyphosphate or a water-soluble salt thereof, other than a monofluorophosphate or a tripolyphosphate, having average chain length of at least 4; where weight ratio of the organic phosphate to inorganic phosphate is 1:10 to 1:0.05, and,

(iii) a pigment having a hue angle, h, in the CIELAB system of from 220 to 320 degrees wherein said composition is a toothpaste, said organic polyphosphate is phytic acid and said inorganic polyphosphate is sodium hexametaphosphate.

In the context of the invention, the "water activity" (a) of the composition is defined as a=p/p o where p is the measured partial pressure of the solution and po is the partial pressure of distilled deionised water. Unless stated otherwise, all water activities are quoted are at ambient temperature. Further references to water activity (or relative humidity, where relative humidity (RH) = 100 a) can be found in Morris, C. and Leech, R., "Natural and Physical Preservative Systems", Curry, J. "Water Activities and Preservatives", Cosmet. Toilet. 100, 53-55, and Christian, J.H.B., "Reduced Water Activity". In:Silliker, J.H. (ed) "Microbial ecology of Foods", vol. 1, Academic Press, New York, pp170-192.

Compositions according to the invention preferably have a water activity of not greater than 0.82, preferably less than 0.7 to 0.81, more preferably less than 0.74 to 0.80.

Yield stress is useful a rheological indicator of consumer perception if the toothpaste appears to be too runny and may be perceived to be less effective with inappropriate sensory. On application of a stress the toothpaste sample behaves as an elastic solid at lower values resisting the flow. Further on reaching a critical value defined as yield stress, the sample starts to flow and the viscosity falls rapidly. Yield stress has been measured by stress sweep measurements using an Anton Paar rheometer with a cone and plate geometry. All measurements were made at 25°C.

It is preferred that yield stress of the composition of the invention is from 250 to 500 Pa.

## Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

"Oral care composition" refers to a composition for which the intended use can include oral care, oral hygiene, or oral appearance, or for which, the intended method of use can comprise administration to the oral cavity. In some embodiments, an oral care composition is not intentionally swallowed, but is rather retained in the oral cavity for a time sufficient to affect the intended utility. The oral care compositions as disclosed herein may be used in nonhuman mammals such as companion animals (e.g., dogs and cats), as well as by humans. In some embodiments, the oral care compositions as disclosed herein are used by humans.

pH profile

The oral care composition of the invention provides a pH of a solution of 1 wt.% of the oral care composition in water as measured at 25°C of from 6.0 to 10.0, preferably from 6.5 to 9.5, more preferably 6.5 to 9.0, even more preferably from 7.0 to 8.0.

Organic polyphosphate

Oral care compositions of the invention comprise an organic polyphosphate or a water-soluble salt thereof, said polyphosphate or said salt having average chain length of at least 4. It is preferred that the composition comprises 0.01 to 5 wt% organic polyphosphate, more preferably 0.05 to 4 wt%, further preferably 0.15 to 3 wt% and most preferably 0.15 to 2 wt%. It is preferred that the organic polyphosphate is phytic acid. In this case, it is in the acid form. Phytic acid, also known as myo-inositol hexaphosphate, or inositol hexaphosphoric acid, is a biodegradable chelating agent in liquid form with chelating performance comparable to that of EDTA. Herein, the term "phytate" includes phytic acid and its salts as well as the other polyphosphorylated inositol compounds.

When the organic polyphosphate is in the form of water-soluble salt, it preferably is an alkali metal salt or an alkaline earth metal salt. Preferably the salt is at least one of sodium phytate, potassium phytate, magnesium phytate, calcium phytate, stannous phytate, zinc phytate, copper phytate or ferrum phytate.

Inorganic polyphosphate

Oral care compositions of the invention comprise an inorganic polyphosphate or a water-soluble salt thereof, having average chain length of at least 4. It is preferred that the composition comprises 0.1 to 5 wt% inorganic polyphosphate, more preferably 0.5 to 4 wt%, further preferably 0.5 to 3 wt% and most preferably 0.5 to 2 wt%.

It is particularly preferred that the inorganic phosphate is hexametaphosphate. More particularly it is sodium hexametaphosphate. It has the structure:

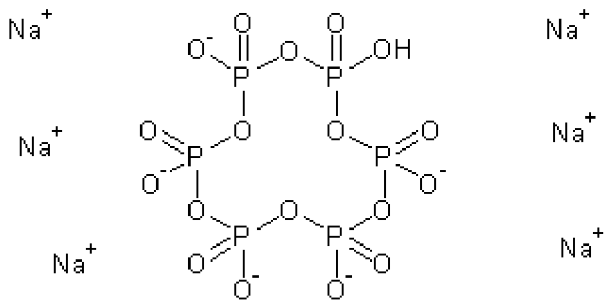

The inorganic polyphosphate or a water-soluble salt thereof is other than a monofluorophosphate or a tripolyphosphate.

The weight ratio of the organic phosphate to inorganic phosphate is 1:10 to 1:0.05, more preferably 1:7 to 1:0.1.

The pigment

The composition also comprises a pigment. The pigment according to the invention is a shade/material which is insoluble in the relevant medium, at the relevant temperature. This is in contrast to dyes which are soluble. The term "soluble", as used herein, means a source that dissolves in water to give a solution with a concentration of at least 0.1 moles per litre. The term "relevant medium", as used herein, refers to human saliva, the liquid medium in which the composition is used, at the temperature of the oral cavity during brushing of the teeth, i.e., up to 37°C. The relevant medium may also be water and the relevant temperature to be 25°C. The only limitation with respect to the pigment is that the same is suitable for use in the mouth.

The pigment has a hue angle, h, in the CIELAB system of from 220 to 320 degrees, more preferably from 250 to 290 degrees. A detailed description of hue angles may be found on p57 of Colour Chemistry 3rd edition by H. Zollinger published by Wiley-VCH. Preferably the pigment is violet or blue, more preferably the pigment is selected from one or more of those listed in the Colour Index International as pigment blue 1 through to pigment 83 and pigment violet 1 through to pigment violet 56. Other suitable pigments are pigment ultramarine blue and ultramarine violet. While the preferred pigment is blue or violet, the same effect may be achieved through mixing pigments outside of this hue angle range. For example, such a hue angle may also be obtained by mixing a red and green-blue pigment to yield a blue or violet shaded

pigment.

It is particularly preferred that the pigment is a blue pigment. Some examples of blue pigments suitable for use in this invention include inorganic blue pigments such as iron blue (C.I. 77510) and ultramarine blue (C.I. 77007). A preferred class of blue pigments suitable for use in the invention are organic blue pigments such as phthalocyanine blue pigments. Phthalocyanines are organometallic compounds containing four symmetrically arranged isoindole rings connected in a 16-membered ring linking with alternate carbon and nitrogen atoms. Most phthalocyanines contains a central, coordinated metal ion such as copper. Copper phthalocyanines have the basis structure:

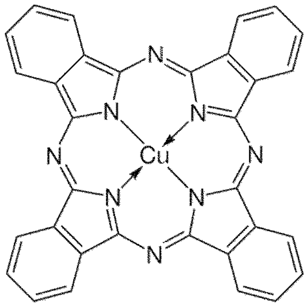

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule. The following pigments are illustrative phthalocyanine blue pigments preferably included in the composition according to the invention:

| C.I. Generic Name | C.I. Constitution Number | Crystal modification; Type | Number of halogen atoms |
|---|---|---|---|
| Pigment Blue 15 | 74160 | Alpha | -- |
| Pigment Blue 15:1 | 74160 | alpha; phase stabilised | 0.5-1 Cl |
| Pigment Blue 15:2 | 74160 | alpha; phase & flocculation stabilised | 0.5-1 Cl |
| Pigment Blue 15:3 | 74160 | Beta | -- |
| Pigment Blue 15:4 | 74160 | beta; flocculation stabilised | -- |
| Pigment Blue 15:6 | 74160 | Epsilon | -- |
| Pigment Blue 16 | 74100 | gamma; metal-free | -- |

It is especially preferred that the pigment is phthalocyanine blue pigment selected from alpha copper phthalocyanines Pigment Blue 15, Pigment Blue 15:1, Pigment 15:2 and mixtures thereof. Most preferably the pigment is Pigment Blue 15:1. A commercially available example is Cosmenyl Blue A4R from Clariant.

The pigment suitable for use in this invention may also be mixtures of any of the above described materials.

The oral care composition of the present invention typically comprises from 0.001 to 1% by weight of the pigment, more preferably from 0.01 to 0.5%, and most preferably from 0.02 to 0.2%, based on total weight of the oral care composition and including all ranges subsumed therein.

Further ingredients

The oral care composition of the invention preferably comprises one or more of the ingredients discussed hereinafter. The exact ingredients and the dosage (wt%) would depend on the nature and type of the oral care composition. For example, while a toothpaste invariably comprises an abrasive, a mouthwash does not need any abrasive. It is preferred that the oral care composition is a toothpaste.

When said composition is a toothpaste, it preferably comprises 20 to 60 wt% of one or more humectants.

Humectants are generally included in toothpastes for a soft a supple mouth feel. Humectants also reduce the tendency of toothpastes to lose moisture. It is preferred that the humectant is at least one of glycerine, sorbitol, maltitol and

xylitol. Sorbitol is available as a 70% aqueous solution. Preferably the compositions comprise glycerine and sorbitol for a lubricated mouth feel, but their cumulative levels do not exceed the disclosed upper limit. Lower humectant content provides an effective way to reduce the cost of the product.

When the composition of the invention is a toothpaste, it preferably comprises an abrasive. The primary function of cleaning agents or abrasives is to provide the necessary cleaning and stain removal to the toothpaste formulation. Typically, cleaning is directly correlated to the abrasivity of silica on tooth dentin and measured by RDA. In other words, the higher the RDA, the higher the abrasivity. Even though this is typically true, some of our cleaning grades can provide high cleaning efficiency at reduced level of abrasion when compared with competitive cleaning grades.

In one aspect the abrasive is silica. Such toothpastes are usually transparent or translucent gels. The term gel indicates physical appearance of the compositions. The gel compositions are transparent to light, i.e., they allow visible light to pass through. Clear gel toothpastes are popular with consumers. When the oral care composition of the invention is a toothpaste in the form of a gel, it is preferred that the compositions do not comprise more than 5 wt%, preferably more than 2 wt% and more preferably more than 1 wt% calcium-based abrasive. That might affect the transparent nature of the compositions.

On the other hand, there are some other types of dentifrice compositions that are not gels but are opaque. That is because such compositions usually contain opacifying ingredients such as chalk or other calcium-based abrasives.

Preferably the composition comprises 5 to 20 wt% silica. Further preferably the composition the silica consists of medium- and high-abrasive silica, where medium-abrasive silica is in majority. Their relative proportions may vary. Preferably the abrasive silica is low refractive index silica with an apparent refractive index in the range of 1.41 to 1.47, preferably 1.435 to 1.445, preferably having a weight mean particle size of between 5 and 15 μm, a BET (nitrogen) surface area of between 10 and 100 $m^2/g$ and an oil absorption of about 70 to 150 $cm^3/100$ g. Examples of suitable low refractive index abrasive silicas having an R.I. of between 1.435 and 1.445 are Tixosil 63 and 73 ex Rhone Poulenc; Sident 10 ex Degussa; Zeodent 113 ex Zeofinn; Sorbosil AC 77 ex Ineos having an R.I. of approximately 1.440. Other examples include the Sylodent® and Syloblanc® range of abrasive silicas from Grace.

Further preferably the oral care compositions comprise 2 to 15 wt% thickening silica. The primary function of thickening silica is to build viscosity, without increasing the level of abrasion (RDA) in the overall formulation. Also, they can be used as texturing agents in formulations that use alternative abrasives other than silica. Our grades of thickening silicas can be categorized by their physical characteristics. These physical characteristics have effect on the rheological properties of the formulation. For example, and perhaps more relevantly, the higher the oil absorption of the thickener, the higher the viscosity imparted to the formulation. In addition to having a substantial impact on the viscosity of the toothpaste system, the thickening silica also plays a role in the mouthfeel of the paste. If the level of the cleaning agent in the system is low, than the formulator may wish to use a less efficient thickener to achieve an overall solids level consistent with the desired mouthfeel. In general, the paste formulation should include at least 15% up to 30% total silica concentration. The split of this percentage between thickener and cleaning agent depends on the choice of silicas, desired cleaning level.

This is especially the case when the compositions comprise silica as the abrasive. A wide variety of thickening silicas are commercially available to choose from. When present, preferred thickening silicas include AEROSIL® T series from Degussa or the CAB-O-SIL® series from Cabot Corporation, silica gels such as the SYLODENT® or SYLOX® series from W. R. Grace & Co or precipitated silica such as ZEOTHIX® 265 from J. M. Huber Corporation. Useful silica thickeners also include ZEODENT® 165, ZEODENT® 163 and/or 167 and ZEOFREE® 153, 177, and/or 265 silicas, all available from J. M. Huber Corporation. Other preferred thickening silicas include MFIL®, MFIL®-P (From Madhu Silica), SIDENT® 22 S and AEROSIL® 200 (Ex. Evonik Industries), SYLODENT® and PERKASIL® thickening silicas from WR Grace & Company and Tixosil® 43 and 331 from Rhodia, synthetic finely divided pyrogenic silica such as those sold under the trademarks SYLOID® 244, SYLOID® 266 and AEROSIL® D-200.

Alternatively, the oral care compositions of the invention comprise calcium -based abrasive. It is particularly preferred abrasive is fine ground natural chalk (FGNC). It is obtained from limestone or marble. FGNC may also be modified chemically or physically by coating during or after milling by heat treatment. Typical coating materials include magnesium stearate and oleate. The morphology of FGNC may also be modified during the milling process by using different milling techniques, for example, ball milling, air-classifier milling or spiral jet milling. FGNC can be used as the sole calcium containing abrasive. However, FGNC can also be used with other calcium containing abrasives to balance the abrasion.

Other preferred calcium-based abrasives include dicalcium phosphate (DCP), calcium pyrophosphate and precipitated calcium carbonate (PCC). When a combination of Calcium-based abrasives is used, it is preferred that FGNC is 35 to 100 %, more preferably 75 to 100 % and especially from 95 to 100 % of the total abrasive. In such cases, the balance, most preferably, is PCC.

Other abrasives can also be used depending upon the intended degree of abrasion and the composition of the paste. These include synthetic abrasive polishing agents such as amorphous precipitated silica and silica gels. Other abrasives include magnesium carbonate, sodium metaphosphate, potassium metaphosphate, zirconium silicate, potassium metaphosphate, magnesium orthophosphate, tricalcium phosphate, magnesium orthophosphate, trimagnesium phosphate, aluminum silicate, zirconium silicate and perlite.

Surfactants

Oral care compositions, especially toothpastes generally contain a surfactant, also commonly referred to as sudsing agent. Suitable surfactants are those which are reasonably stable and provide foam throughout a wider pH range. It is preferred that compositions comprise an anionic surfactant.

Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate and sodium coconut monoglyceride sulfonates are examples of anionic surfactants of this type.

Preferably the oral care compositions comprise 0.25 to 12 wt%, more preferably from 0.5 to 8 wt%, and most preferably from 1 to about 6 wt% anionic surfactants.

Some anionic surfactants, in particular, sodium lauryl sulphate itself have antibacterial effect. Such action provides some degree of instant antibacterial effect. However, this effect is generally very short-lived. Other surfactants like nonionic, amphoteric or zwitterionic surfactants may also be included.

Nonionic surfactants can be broadly defined as compounds produced by the condensation of alkylene oxide groups (hydrophilic in nature) with an organic hydrophobic compound which may be aliphatic or alkyl- aromatic in nature.

Examples of suitable nonionic surfactants include poloxamers (sold under trade name PLURONIC®), polyoxyethylene, polyoxyethylene sorbitan esters (sold under trade name TWEENS®), POLYOXYL® 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and mixtures of such materials.

Further preferably the oral care compositions of the invention comprises a thickening system which comprises thickening silica and at least one of carboxymethyl cellulose, xanthan gum or guar gum. Other binders and thickeners such as sodium carboxymethylcellulose, xanthan gum, gum arabic may also be included, as well as synthetic polymers such as polyacrylates and carboxyvinyl polymers such as Carbopol®. It is preferred that dentifrice compositions of the invention comprise a thickening system which comprises thickening silica and at least one of carboxymethyl cellulose, xanthan gum or guar gum.

Preferred oral care compositions comprise a binder, which lends a good structure to the paste. Cellulosic binders are especially preferred. Preferred cellulosic binders include cellulose ethers, which include hydroxyethyl cellulose (HEC), hydroxypropyl cellulose (HPC), ethylhydroxyethyl cellulose (EHEC), carboxymethyl cellulose (CMC), carboxymethylhydroxyethyl cellulose (CMHEC), hydroxypropylhydroxyethyl cellulose (HPHEC), methyl cellulose (MC), methylhydroxypropyl cellulose (MHPC), methylhydroxyethyl cellulose (MHEC), carboxymethylmethyl cellulose (CMMC), hydrophobically modified carboxymethyl cellulose (HMCMC), hydrophobically modified hydroxyethyl cellulose (HMHEC), hydrophobically modified hydroxypropyl cellulose (HMHPC), hydrophobically modified ethylhydroxyethyl cellulose (HMEHEC), hydrophobically modified carboxymethylhydroxyethyl cellulose (HMCMHEC), hydrophobically modified hydroxypropylhydroxyethyl cellulose (HMHPHEC), hydrophobically modified methyl cellulose (HMMC), hydrophobically modified methylhydroxypropyl cellulose (HMMHPC), hydrophobically modified methylhydroxyethyl cellulose (HMMHEC), and hydrophobically modified carboxymethylmethyl cellulose (HMCMMC).

Other cellulosic binders include cationic hydroxyethyl cellulose (cationic HEC), cationic hydrophobically modified hydroxyethyl cellulose (cationic HMHEC) and microcrystalline cellulose.

A highly preferred binder is Sodium carboxymethyl cellulose (SCMC). Particularly preferred sodium carboxymethyl celluloses include those with degree of substitution of from 0.6 to 0.99, preferably from 0.7 to 0.95.

Preferred toothpaste compositions may also include one or more other thickening agents such as carboxyvinyl polymers which include carbomers which are commercially available from B. F. Goodrich as the CARBOPOL® series, including CARBOPOL® 934, 940, 941 and 956.

Other preferred grades include acrylates/$C_{10}$-$_{30}$ alkyl acrylate cross polymers which are commercially available as ULTREZ® 21, PEMULEN® TR-1, and PEMULEN® TR-2, from Noveon Corporation. Preferred compositions can include 0.05 to 10 wt%, more preferably 0.1 to 5 wt%, and even more preferably 0.25 to about 4 wt% of other thickening agents.

The oral care compositions of the invention may contain optional further ingredients such as cosmetically acceptable carriers like starch or sucrose.

Preferred compositions can have alkali metal silicate. The alkali metal is sodium or potassium, preferably sodium. Sodium silicate is generally available as 10 to 40 % aqueous solution, most common being 30 % solution. Sodium silicate is available as neutral sodium silicate or alkaline sodium silicate. Preferred toothpastes have neutral sodium

silicate. Sodium silicate is available with varying ratios of $Na_2O$: $SiO_2$.

Sodium silicate with $Na_2O$: $SiO_2$ ratio in the range of 3.0 to 3.8 is preferred, more highly preferred range being 3.25 to 3.5. Preferred toothpastes include 0.1 to 5 wt% silicate (on dry weight basis). Thus, a 30 % solution of sodium silicate is added to the composition in an amount in the range of 0.3 to 16 wt%.

Flavours such as peppermint and spearmint oils may also be included, as well as preservatives, colouring agents, pH adjusting agents, sweetening agents and so on. Suitable flavoring components include oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, ethyl vanillin, heliotropine, 4-cis-heptenal, diacetyl, methyl-para-tert-butyl phenyl acetate, and mixtures thereof. Coolants may also be part of the flavor system. Preferred coolants are the paramenthane carboxyamide agents such as N-ethyl-p-menthan-3-carboxamide (known commercially as "WS-3®") and mixtures thereof. The flavour is generally from 0.001 to 5 wt%.

Anti-caries agents such as sodium- and stannous fluoride, aminefluorides, monosodiumfluorophosphate, casein, plaque buffers such as urea, pyruvates, arginine, small peptides, calcium lactate, calcium glycerophosphate, strontium polyacrylates may also be included. A preferred anti-caries agent is sodium monofluorophosphate. Other optional ingredients include vitamins such as Vitamin C, and plant extracts. Desensitising agents such as potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the oral care compositions may comprise anti-calculus agents such a alkali metal pyrophosphates, hypophosphite-containing polymers, organic phosphonates and phosphor-citrates.

Other optional ingredients that may be included are e.g. bleaching agents such as peroxy compounds e.g. potassium peroxy diphosphate, effervescing systems such as sodium bicarbonate/citric acid systems and colour change systems.

The compositions of the invention may also contain coloured particles suspended therein, e.g. coloured silica agglomerates or other coloured particles to impart a "speckled" appearance to the dentifrices. The oral care compositions may also contain stripes of a coloured paste, to provide for a visually clear gel-type dentifrice with coloured stripes or a separate coloured phase

Toothpastes with calcium containing abrasives especially chalk are prone to bacterial growth. Certain preservatives, e.g., methyl, ethyl, butyl, propyl and isopropyl esters of parahydroxybenzoic acid may be particularly useful against bacterial growth. A mixture of methyl, ethyl, butyl and propyl esters of parahydroxybenzoic acid is particularly preferred.

The activity of this mixture can be enhanced by adding phenoxyethanol. Formaldehyde and dimethyl hydantoin are other preferred preservatives. Preservatives are generally included at 0.005 to 0.8 wt%.

The oral care composition of the present invention is prepared by conventional methods of making oral care compositions. Such methods include mixing the ingredients under moderate shear and atmospheric pressure.

Typically, the composition is packaged.

In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may be used daily, for example for use by an individual once, twice or three times per day.

Therapeutic treatments can be summarized as being measures directed to the maintenance (prophlyaxis) or restoration (therapy) of health. 'Therapy' is concerned with bringing a body from a pathological state back to its normal, healthy state and with preventing a pathological state. Treatment by therapy is defined as any treatment which is designed to cure, alleviate, remove or lessen the symptoms of, or prevent or reduce the possibility of contracting any disorder or malfunction of the human or animal body.

<u>The packaged product</u>

In accordance with another aspect is disclosed a packaged product comprising the aqueous gel composition of the invention. The gel composition is preferably packaged in a collapsible tube such that it becomes easy to dispense a small amount without much dexterity. Alternatively, the composition is packaged in another type of container, such as a bottle or a jar, preferably with suitable means for dispensing a known quantity of the composition. The pack may be further packed in a suitable secondary package like a carton or a box, preferably with useful information about the contents and instructions for use.

The invention will now be further described with reference to the following non-limiting examples.

**Examples**

Example 1: $\Delta E$ of compositions inside and outside the invention

The experiments were performed on Hydroxyapatite (HAP) discs because they are most suited for this purpose. In a first step the discs immersed in an aqueous solution of the toothpaste composition. Then the discs were lightly brushed using a toothbrush to simulate brushing of teeth. The discs were then rinsed with clean water. The disc were then stained by immersing them for 10 minutes in a 2% aqueous solution of tea. This step of stain was repeated two more times. All the discs turned dark brown. At the stage the L*a*b* values were determined. Absorbance measurement over the entire visible color spectrum are obtained using the CIELAB color scale (Commission International de L'Eclairage, 1978 and 1986) with a SpectroShade® Micro spectrophotometer (MHT, Italy). This scale quantifies color according to three parameters, L* (lightness-darkness); a* (red-green); and b* (yellow-blue) with increasing L* indicating a more aesthetically pleasing and desirable colour (whiter teeth). The L*, a*, b* values of each HAP disc were measured CM-2600d Spectrophotometer (Spectral) from Konica Minolta Sensing value was calculated based on L*, a*, b* values measured using CM-2600d Spectrophotometer (Spectral) from Konica Minolta Sensing with the following formula:

$$\Delta E = \sqrt{\Delta L^2 + \Delta a^2 + \Delta b^2}$$

$\Delta L$ = L1-L0, $\Delta a$ = a1 - a0, $\Delta b$ = b1 - b0, where L1, a1 and b1 are the color index of HAP discs treated by samples, and L0, a0 and b0 are the color index of blank HAP discs.

After the initial values were recorded, the discs immersed in an aqueous solution of the toothpaste composition. Then the discs were lightly brushed using a toothbrush to simulate brushing of teeth. The discs were then rinsed with clean water and the L*a*b* values were determined again. At this stage the $\Delta E$ cal calculated was reported as anti-stain efficacy.

This stained were then cleaned by immersing the disc in an aqueous solution of the toothpaste composition. Then the discs were lightly brushed using a toothbrush to simulate brushing of teeth. The discs were then rinsed with clean water and L*a*b* values of HAP discs were determined again. This was termed as the first cycle of treatment after which the absorbance measurements were repeated to determine the change in L* value so that difference between the initial L* value and the corresponding value after the first cycle of treatment could be determined.

After recording the L* value, the series of steps starting from (re) application of the selected composition and ending with the measurement of L*, were repeated. This was done until there was data pertaining to four cycles of treatment. Details are shown in Table 1.

**Table 1**

| Ingredient | Refence number/wt% | | | |
|---|---|---|---|---|
| | Ex1 | Ex2 | Ex3 | Ex4 |
| Sorbitol (70%) | 45 | 45 | 45 | 45 |
| Thickening Silica | 8 | 8 | 8 | 8 |
| Abrasive Silica (med) | 8.5 | 8.5 | 8.5 | 8.5 |
| Abrasive Silica (high) | 1.65 | 1.65 | 1.65 | 1.65 |
| SLS | 1.77 | 1.77 | 1.77 | 1.77 |
| Sodium Phytate | -- | 2.5 | 5 | -- |
| SHMP | -- | 2.5 | -- | 5 |
| SCMC 9H | 0.8 | 0.8 | 0.8 | 0.8 |
| Blue Covarine | -- | 0.025 | 0.025 | 0.025 |
| NaOH solution (10%) | Adjust to pH~7.00 | Adjust to pH~7.00 | Adjust to pH~7.00 | Adjust to pH~7.00 |
| Water and others | To 100 wt% | To 100 wt% | To 100 wt% | To 100 wt% |

Ex1 is control. Ex2 is according to the invention. Ex3 and Ex4 are comparative examples.

The important observations and data is shown in table 2.

**Table 2**

| | | ΔE-pretreatment | ΔE-staining | ΔE-cleaning |
|---|---|---|---|---|
| Ex-4 | 5% HMP + BC | 2.79 | 11.56 | 9.39 |
| Ex-3 | 5% Phytate + BC | 4.27 | 9.26 | 8.59 |
| Ex-2 | HMP + Phytate + BC | 3.20 | 7.72 | 6.58 |
| Ex-1 | Control | 3.95 | 15.51 | 13.65 |
| | | b*-pretreatment | b*-staining | b*-cleaning |
| Ex-4 | 5% HMP | 1.13 | 9.97 | 7.30 |
| Ex-3 | 5% Phytate | 1.29 | 7.56 | 5.40 |
| Ex-2 | 5% HMP+Phytate | 1.06 | 6.76 | 4.68 |
| Ex-1 | Control | 1.74 | 12.68 | 10.70 |
| Note: BC=Blue covarine. | | | | |

The data in Table 2 indicates that combination of phytate, hexametaphosphate and the dye, blue covarine, is synergistic and provides superior whitening demonstrated by lower ΔE and b* values.

## Claims

**1.** An oral care composition comprising:

(i) an organic polyphosphate or a water-soluble salt thereof, said polyphosphate or said salt having average chain length of at least 4;
(ii) an inorganic polyphosphate or a water-soluble salt thereof, other than a monofluorophosphate or a tripolyphosphate, having average chain length of at least 4; where weight ratio of the organic phosphate to inorganic phosphate is 1:10 to 1:0.05, and,
(iii) a pigment having a hue angle, h, in the CIELAB system of from 220 to 320 degrees

wherein said composition is a toothpaste, said organic polyphosphate is phytic acid and said inorganic polyphosphate is sodium hexametaphosphate.

**2.** An oral care composition as claimed in claim 1 wherein water activity of said composition is not greater than 0.82.

**3.** A composition as claimed in claim 1 or 2 wherein at 25°C, the yield stress of said composition is from 250 to 500 Pa.

**4.** A composition as claimed in any of claims 1 to 3 wherein said weight ratio of the organic phosphate to inorganic phosphate is 1:7 to 1:0.1.

**5.** A composition as claimed in any of claims 1 to 4 wherein said composition comprises 0.01 to 5 wt% organic polyphosphate.

**6.** A composition as claimed in any of claims 1 to 5 wherein said composition comprises 0.1 to 5 wt% of said inorganic polyphosphate.

**7.** A composition as claimed in any of claims 1 to 6 wherein said water-soluble salt of organic polyphosphate is an alkali metal salt or an alkaline earth metal salt.

**8.** A composition as claimed in any of claims 1 to 7 wherein said composition comprises 6 to 20 wt% abrasive.

**9.** A composition as claimed in claim 8 wherein said abrasive is silica.

**10.** A composition as claimed in any of claims 1 to 9 wherein said composition comprises 10 to 45 wt% humectant.

11. A composition as claimed in any of claims 1 to 10 wherein said hue angle, h, in the CIELAB system is 250 to 290 degrees.

## Patentansprüche

1. Mundpflegezusammensetzung, umfassend:

   (i) ein organisches Polyphosphat oder ein wasserlösliches Salz davon, wobei das Polyphosphat oder das Salz eine durchschnittliche Kettenlänge von mindestens 4 aufweist;
   (ii) ein anorganisches Polyphosphat oder ein wasserlösliches Salz davon, das kein Monofluorphosphat oder Tripolyphosphat ist, mit einer durchschnittlichen Kettenlänge von mindestens 4, wobei das Gewichtsverhältnis von organischem Phosphat zu anorganischem Phosphat 1:10 bis 1:0,05 beträgt, und
   (iii) ein Pigment mit einem Farbtonwinkel h im CIELAB-System von 220 bis 320 Grad, wobei die Zusammensetzung eine Zahnpasta ist, das organische Polyphosphat Phytinsäure ist und das anorganische Polyphosphat Natriumhexametaphosphat ist.

2. Mundpflegezusammensetzung, wie im Anspruch 1 beansprucht, wobei die Wasseraktivität der Zusammensetzung nicht größer als 0,82 ist.

3. Zusammensetzung, wie im Anspruch 1 oder 2 beansprucht, wobei die Fließgrenze der Zusammensetzung bei 25°C zwischen 250 und 500 Pa liegt.

4. Zusammensetzung, wie in einem der Ansprüche 1 bis 3 beansprucht, wobei das Gewichtsverhältnis von organischem Phosphat zu anorganischem Phosphat 1:7 bis 1:0,1 beträgt.

5. Zusammensetzung, wie in einem der Ansprüche 1 bis 4 beansprucht, wobei die Zusammensetzung 0,01 bis 5 Gew.-% organisches Polyphosphat umfasst.

6. Zusammensetzung, wie in einem der Ansprüche 1 bis 5 beansprucht, wobei die Zusammensetzung 0,1 bis 5 Gew.-% des anorganischen Polyphosphats umfasst.

7. Zusammensetzung, wie in einem der Ansprüche 1 bis 6 beansprucht, wobei das wasserlösliche Salz des organischen Polyphosphats ein Alkalimetallsalz oder ein Erdalkalimetallsalz ist.

8. Zusammensetzung, wie in irgendeinem der Ansprüche 1 bis 7 beansprucht, wobei die Zusammensetzung 6 bis 20 Gew.-% Schleifmittel umfasst.

9. Zusammensetzung, wie im Anspruch 8 beansprucht, wobei das Schleifmittel Siliciumdioxid ist.

10. Zusammensetzung, wie in einem der Ansprüche 1 bis 9 beansprucht, wobei die Zusammensetzung 10 bis 45 Gew.-% Feuchthaltemittel umfasst.

11. Zusammensetzung, wie in einem der Ansprüche 1 bis 10 beansprucht, wobei der Farbtonwinkel h im CIELAB-System 250 bis 290 Grad beträgt.

## Revendications

1. Composition de soins bucco-dentaires comprenant :

   (i) un polyphosphate organique ou un sel de celui-ci soluble dans l'eau, ledit polyphosphate ou ledit sel ayant une longueur de chaîne moyenne d'au moins 4 ;
   (ii) un polyphosphate inorganique ou un sel de celui-ci soluble dans l'eau, autre qu'un monofluorophosphate ou qu'un tripolyphosphate, ayant une longueur de chaîne moyenne d'au moins 4 ; où le rapport en poids du phosphate organique au phosphate inorganique est de 1/10 à 1/0,05, et
   (iii) un pigment ayant un angle de teinte, h, dans le système CIELAB, de 220 à 320 degrés,

dans laquelle ladite composition est une pâte dentifrice, ledit polyphosphate organique est l'acide phytique et ledit polyphosphate inorganique est l'hexamétaphosphate de sodium.

**2.** Composition de soins bucco-dentaires selon la revendication 1, dans laquelle l'activité de l'eau de ladite composition n'est pas supérieure à 0,82.

**3.** Composition selon la revendication 1 ou 2, dans laquelle, à 25 °C, le seuil d'écoulement de ladite composition est de 250 à 500 Pa.

**4.** Composition selon l'une quelconque des revendications 1 à 3, dans laquelle ledit rapport en poids du phosphate organique au phosphate inorganique est de 1/7 à 1/0,1.

**5.** Composition selon l'une quelconque des revendications 1 à 4, dans laquelle ladite composition comprend 0,01 à 5 % en poids de polyphosphate organique.

**6.** Composition selon l'une quelconque des revendications 1 à 5, dans laquelle ladite composition comprend 0,1 à 5 % en poids dudit polyphosphate inorganique.

**7.** Composition selon l'une quelconque des revendications 1 à 6, dans laquelle ledit sel soluble dans l'eau de polyphosphate organique est un sel de métal alcalin ou un sel de métal alcalino-terreux.

**8.** Composition selon l'une quelconque des revendications 1 à 7, dans laquelle ladite composition comprend 6 à 20 % en poids d'abrasif.

**9.** Composition selon la revendication 8, dans laquelle ledit abrasif est la silice.

**10.** Composition selon l'une quelconque des revendications 1 à 9, dans laquelle ledit composition comprend 10 à 45 % en poids d'humectant.

**11.** Composition selon l'une quelconque des revendications 1 à 10, dans laquelle l'angle de teinte, h, dans le système CIELAB, est de 250 à 290 degrés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020135952 A1
- WO 2021063581 A1
- US 20160331652 A1
- WO 07111616 A1
- US 20200046621 A1
- US 2015289961 A1
- EP 2741732 A1
- EP 1935395 A1
- US 2007122358 A1
- WO 2016099544 A1
- CN 109223642 A


### Non-patent literature cited in the description


- **MORRIS, C.** ; **LEECH, R.** *Natural and Physical Preservative Systems*
- **CURRY, J.** Water Activities and Preservatives. *Cosmet. Toilet.*, vol. 100, 53-55
- Reduced Water Activity. **CHRISTIAN, J.H.B**. Microbial ecology of Foods. Academic Press, vol. 1, 170-192
- **H. ZOLLINGER**. Colour Chemistry. Wiley-VCH, 57